# EUROPEAN PATENT SPECIFICATION

(11) **EP 1 090 618 B1**
(45) Date of publication and mention of the grant of the patent: **16.03.2005**
(21) Application number: 00308877.0
(22) Date of filing: 09.10.2000
(51) Int. Cl.: A61F 13/56

(54) **Diaper fastener**
Windelverschluss
Système de fermeture de couche-culotte

(30) Priority: 08.10.1999 FR 9912591
(43) Date of publication of application: 11.04.2001
(73) Proprietor: YKK EUROPE LTD., London NW1 3BG (GB)
(72) Inventor: Vo, The Hung, 59471 Seclin Cedex (FR)
(74) Representative: Luckhurst, Anthony Henry William

(56) References cited:
- WO-A-97/02795
- GB-A- 1 377 541
- US-A- 5 901 419

## Description

The present invention relates to provide a nappy incorporating a fastener, and in particular a low cost fastener. The term nappy embraces nappies which are worn by babies and young children, but this invention may also have utility with incontinence pants worn by older people.

Re-usable nappies or "Terrys" are typically fastened on the wearer by means of a safety pin, but designs using hook and loop surface fasteners are known from the patent literature. Disposable nappies have long used touch sensitive adhesive tapes to fasten the diaper, but in recent years surface fasteners of the hook and loop type have been used. The hook and loop type fastener overcomes the drawback of contamination of the surface of the adhesive of the adhesive type fastener, with powder, cream, etc., when fitting the nappy, and can also be reused more times than the adhesive type fastener. When fastened, both types of fastener, adhesive and hook and loop type, fix the orientation of the nappy parts on which the are fastened.

One embodiment of the present invention provides a nappy having a mechanical fastener as set forth in the accompanying claim 1.

Document US-A-5 901 419 shows a nappy having a fastener according to the preamble of claim 1.

By providing a fastener with relatively movable parts, the nappy is more comfortable for the wearer.

The fastener is simple to make and of low cost.

Other aspects and preferred features of the invention will be apparent from the following description and the accompanying claims.

The invention will be further described by way of example, with reference to the accompanying drawing, in which:
Figure 1 is a schematic view of a nappy in accordance with the invention.
Figure 2 is a plan view of a fastener of the nappy of Figure 1, with the parts separated, and
Figure 3 is a cross-section along line 111-111 of Figure 2.

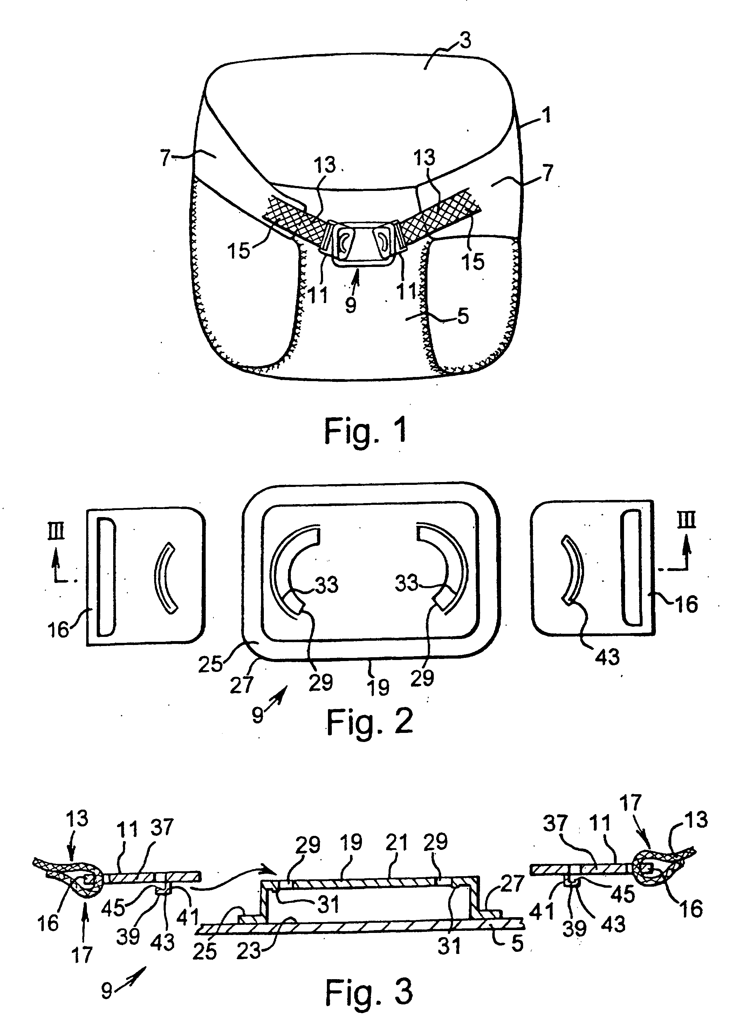

Figure 1 shows schematically a nappy 1. The nappy may be of the type used on babies, but can also be of the type used on older persons for incontinence, etc. The nappy may be a re-usable nappy or a disposable nappy, i.e. one intended to be disposed of after a single use.

The nappy 1 has a rear panel 3 integral with a front panel 5, and wings 7. The nappy is fitted on a wearer by attaching the wings 7 to the front panel 5, as is well known in the art. The nappy 1 utilises a novel type of mechanical fastener 9 to secure the wings 7 to the front panel 5 which allows a range of adjustment or movement of the relative orientation of the wings 7 and panel 5 while the nappy is being worn.

Fastener 9 comprises two male members 11 which are attached to a respective wing 7 by an elastic strap 13. Strap 13 is glued, sewn or welded to the wing 7 at one end 15 and secured around a bar 16 on the male member 11 at the other end 17, for example by sewing or welding the material of the strap 13.

Fastener 9 further comprises a female member 19 which is attached to the front panel 5, for example by stitching, welding or gluing.

It will be appreciated that the manner of attachment of the fastener parts to the nappy will be suited to the weight and type of materials used and the strength required according to the expected use and lifetime of the nappy. Fastener 9 will typically be moulded of plastics material.

Female member 19 has a planar coupling wall 21 which is spaced from the surface 23 of the panel 5 by an L-shaped rim 25. The foot 27 of the rim 25 is attached to surface 23 as indicated above. Wall 21 has two arcuate slots 29 which each receive a respective male member, as will be described hereinafter. A nib 31 is provided along one outer edge 33, on the underside 35 of the wall 19.

Each male member 11 comprises a planar wall 37 and an L-shaped locking tongue 39 which projects below the wall 37. Locking tongue 39 comprises a first arcuate wall 41 which has a radius of curvature close to the radius of curvature of the edge 33 of slot 29, but extends over a shorter arcuate length. A second arcuate wall 43, forming the foot of the L-shape, has a re-entrant nib 45.

To couple a male member 11 to the female member 19, the L-shaped locking tongue 39 is passed into the slot 29 and the male member 11 pulled radially outward in the plane of wall 19 to snap the re-entrant nib 45 behind nib 31.

The elastic straps 13 will apply tension to the coupling to maintain the coupling of the members 11, 19. Male member 11 is able to pivot relative to the female member 19 in the plane of coupling of the members 11, 19, the L-shaped locking tongue 39 sliding in the slot 29.

The clearance between nib 45 and wall 37 may be greater, so that nib 45 does not have to be snapped into position.

Reliance could be placed solely on the elastic straps 13 to maintain engagement between the members 11, 19, and nibs 31, 45 could be omitted.

Thus the fastener 9 allows for changes in the relative orientation of the fastener parts 11, 19 to facilitate coupling of the fastener when applying the nappy to a wearer, and to allow greater comfort in use.

The fastener may incorporate a single member 11, or several. The L-shaped locking tongue 39 may be formed on the member 19, with the co-operating slot 29 being provided on the member 11.

## Claims

1. A nappy having a mechanical fastener (9), the fastener (9) comprising a male member (11) and a female member (19) attached to respective parts (5, 7) of the nappy, the male member (11) being coupled in use to the female member (19), wherein the male member (11) can be pivoted relative to the female member (19) when the members (11, 19) are coupled togheter, **characterised in that** the male member comprises an arcuate locking member (39) which is engaged with a wall (33) of an arcuate slot (29) in the female member (19) to fasten the male and female members (11, 19) together.

2. A nappy as claimed in claim 1, **characterised in that** the locking member 39 is an L-shaped wall which is engaged with the edge of the slot (29).

3. A nappy as claimed in claim 2, **characterised in that** nibs (31, 45) are provided on the locking member (39) and an edge (33) of the slot to maintain engagement between the locking member (39) and the slot (29).

## Patentansprüche

1. Windel mit einem mechanischen Verschluss (9), wobei der Verschluß (9) ein Steckelement (11) und ein Aufnahmeelement (19) aufweist, die an entsprechenden Teilen (5, 7) der Windel befestigt sind, wobei das Steckelement (11) bei Benutzung mit dem Aufnahmeelement (19) verbunden wird, wobei das Steckelement (11) drehbar relativ zum Aufnahmeelement (19) angeordnet ist, wenn die Elemente (11, 19) miteinander verbunden werden, **dadurch gekennzeichnet, daß** das Steckelement ein bogenförmiges Feststellelement (39) aufweist, das mit einer Wand (33) eines bogenförmigen Schlitzes (29) im Aufnahmeelement (19) in Eingriff gebracht wird, um das Steckelement und das Aufnahmeelement (11, 19) aneinander zu befestigen.

2. Windel nach Anspruch 1, **dadurch gekennzeichnet, daß** das Feststellelement (39) eine L-förmige Wand ist, die mit dem Rand des Schlitzes (29) in Eingriff gebracht wird.

3. Windel nach Anspruch 2, **dadurch gekennzeichnet, daß** Enden (31, 45) am Feststellelement (39) und einem Rand (33) des Schlitzes vorhanden sind, um den Eingriff zwischen dem Feststellelement (39) und dem Schlitz (29) aufrechtzuerhalten.

## Revendications

1. Couche-culotte comportant un élément de fixation mécanique (9), l'élément de fixation (9) comprenant un élément mâle (11) et un élément femelle (19) fixés sur des parties respectives (5, 7) de la couche-culotte, l'élément mâle (11) étant accouplé en service à l'élément femelle (19), l'élément mâle (11) pouvant être pivoté par rapport à l'élément femelle (19) lors de l'accouplement des éléments (11, 19), **caractérisée en ce que** l'élément mâle comprend un élément de blocage arqué (39) engagé dans une paroi (33) d'une fente arquée (29) dans l'élément mâle (19) pour fixer les éléments mâle et femelle (11, 19) l'un à l'autre.

2. Couche-culotte selon la revendication 1, **caractérisée en ce que** l'élément de blocage 39 est constitué par une paroi en L engagée dans la fente (29).

3. Couche-culotte selon la revendication 2, **caractérisée en ce que** des crans (31, 45) sont agencés sur l'élément de blocage (39) et un bord (33) de la fente pour maintenir l'engagement entre l'élément de blocage (39) et la fente (29).
